# EUROPÄISCHE PATENTSCHRIFT

(11) **EP 1 318 799 B1**
(45) Veröffentlichungstag und Bekanntmachung des Hinweises auf die Patenterteilung: **03.12.2008**
(21) Anmeldenummer: 01969738.2
(22) Anmeldetag: 19.09.2001
(51) Int. Cl.: A61K 31/122, A61K 9/14, A61K 9/16, A61K 9/20

(54) **MECHANISCH STABILE DARREICHUNGSFORMEN, ENTHALTEND UBICHINONE**
STABLE DOSAGE FORMS CONTAINING UBIQUINONES
FORMES GALENIQUES STABLES CONTENANT DES UBIQUINONES

(30) Priorität: 19.09.2000 DE 10046541
(43) Veröffentlichungstag der Anmeldung: 18.06.2003
(73) Patentinhaber: Abbott GmbH & Co. KG, 65205 Wiesbaden (DE)
(72) Erfinder: ROSENBERG, Jörg, 67158 Ellerstadt (DE); BREITENBACH, Jörg, 68199 Mannheim (DE)
(74) Vertreter: Kinzebach, Werner
(86) Internationale Anmeldenummer: PCT/EP2001/010830
(87) Internationale Veröffentlichungsnummer: WO 2002/024184

(56) Entgegenhaltungen:
- EP-A- 0 240 904
- WO-A-01/62226
- WO-A-98/08490
- US-A- 6 107 276
- DATABASE CA [Online] CHEMICAL ABSTRACTS SERVICE, COLUMBUS, OHIO, US; TATEISHI, KIMIO ET AL: "Solid pharmaceutical preparations with high uniformity" retrieved from STN Database accession no. 89:12171 CA XP002193694 & JP 52 136912 A (EISAI CO., LTD., JAPAN) 16. November 1977 (1977-11-16)

## Beschreibung

Die vorliegende Erfindung betrifft ein Verfahren gemäß Anspruch 1 zur Herstellung stabiler Darreichungsformen von Ubichinonen zur peroralen Verabreichung, enthaltend mindestens einen thermoplastisch verarbeitbaren matrixbildenden Hilfsstoff, wobei die Ubichinone bevorzugt als feste Dispersion in der Hilfsstoffmatrix vorliegen.

Ubichinone sind fettlösliche Substanzen mit dem Grundgerüst des 2,3-Dimethoxy-5-methyl-1,4-benzochinons und einer isoprenoiden Seitenkette mit 1-10 Dihydroisopren-Einheiten (n = 1 bis 10) der allgemeinen Formel (I)

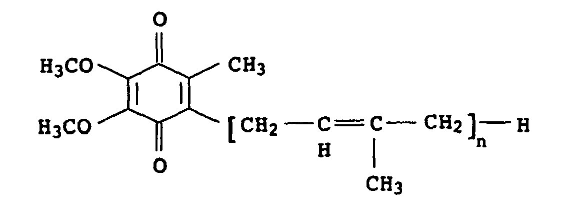

Vor allem Ubichinone mit n = 3 bis n = 5 sind in der Natur relativ weit verbreitet.

Die vorliegende Erfindung betrifft insbesondere das auch als Coenzym Q10 bezeichnete Ubichinon-50 (n = 10). Dabei handelt es sich um eine gelb-orange gefärbte Substanz mit einem Festpunkt von ca. 50°C, die in organischen Lösungsmitteln und Fetten, in Wasser jedoch sehr schwer löslich ist.

Die Ubichinone weisen aufgrund ihrer Struktur allgemein eine sehr schlechte Löslichkeit in Wasser auf, was Probleme hinsichtlich der ausreichenden Bioverfügbarkeit bereitet.

Weiterhin kann der niedrige Schmelzpunkt z.B. des Ubichinon-50 Schwierigkeiten hinsichtlich der Verarbeitung und der Stabilität der Darreichungsformen bereiten bzw. die Wahl einer geeigneten Form einschränken.

Eine Grundvoraussetzung für eine befriedigende Bioverfügbarkeit ist ein ausreichendes Auflösungsvermögen des Wirkstoffs im wäßrigen Milieu des Verdauungstrakts. Die Resorption des wirkstoffs ist nur dann möglich, wenn dieser gelöst vorliegt, da nur gelöste Wirkstoffe die Darmwand passieren können. Im Falle schwer löslicher Wirkstoffe kann dies zu ungenügender Resorption und damit verbundener niedriger Bioverfügbarkeit führen.

Es hat nicht an Versuchen gefehlt, die Bioverfügbarkeit schwer löslicher Wirkstoffe zu verbessern (vgl. R. Voigt; "Pharmazeutische Technologie", Verlag Ullstein Mosby, 7. Auflage, 1993, Seiten 80-85). Insbesondere die Herstellung von Coevaporaten oder sogenannten festen Dispersionen, in denen der Wirkstoff molekulardispers in einer Hilfsstoffmatrix verteilt vorliegen kann, hat sich häufig als vorteilhaft für die Erhöhung der Bioverfügbarkeit erwiesen. Bei der Auflösung der Arzneiform im Körper kann der Wirkstoff aus solchen festen Dispersion direkt und ohne Aufbringen von Solvatationsenergie molekular freigesetzt werden.

Einen positiven Einfluß auf die Bioverfügbarkeit des Wirkstoffs hat die Anwendung von festen Dispersionen aber nur, wenn der Wirkstoff auch schnell resorbierbar ist. Ist der Resorptionsvorgang aber langsam, kommt es zu einer Rekristallisation des schwerlöslichen Wirkstoffs im wäßrigen Milieu des Darmlumens, da bei der Auflösung der Arzneiform eine übersättigte Wirkstofflösung entstehen kann. Aus diesem Grund sind auch mit festen Dispersionen in bestimmten Fällen nur unbefriedigende Bioverfügbarkeiten zu erzielen.

Oft scheitert die ausreichende Resorption des Wirkstoffs auch daran, daß der Wirkstoff zu langsam aus der Darreichungsform, z.B. einer Tablette freigesetzt wird. Die Resorption in die Blutzirkulation findet für den überwiegenden Teil aller Wirkstoffe in den oberen Dünndarm-Abschnitten statt, d.h. relativ kurz nach Passage des Magens. Wirkstoffe, die in diesem Bereich des Dünndarms noch nicht ausreichend solubilisiert wurden, können nur noch begrenzt resorbiert werden.

Zur Erzielung optimaler Resorptionsraten ist es daher entscheidend, insbesondere bei schwer löslichen, leicht kristallisierenden Wirkstoffen eine schnelle und genügend lang andauernde Solubilisierung im wäßrigen Milieu des Verdauungstrakts zu erreichen, ohne daß dabei eine Rekristallisation eintritt.

Die Zugabe von oberflächenaktiven Substanzen zu Formulierungen schwerlöslicher Wirkstoffe ist an sich allgemein bekannt.

Aus der US-A 5,834,472 ist beispielsweise bekannt, daß durch Mitverwendung einer nichtionischen oberflächenaktiven Substanz die Bioverfügbarkeit eines Antifungicides verbessert werden kann.

Da die meisten der oberflächenaktiven Substanzen bei Raumtemperatur aber flüssig oder halbfest sind, werden daher bisher meist flüssig-ölige oder halbfeste Zubereitungen hergestellt, die dann in Hart- oder Weichgelatine-Kapseln abgefüllt werden. Jedoch treten bei Weichgelatine-Kapseln häufig Wechselwirkungen zwischen Hilfsstoffen und der Gelatinehülle der Kapsel auf, die zum vorzeitigen Auslaufen der Kapsel führen.

Auch der Einsatz der oberflächenaktiven Substanzen in Tabletten-Formulierungen ist nicht ohne weiteres möglich, da die flüssigen oder halbfesten oberflächenaktiven Substanzen die Verpreßbarkeit beim konventionellen Tablettierprozeß verhindern, insbesondere dann, wenn zur Solubilisierung des Wirkstoffs größere Mengen an oberflächenaktiven Substanzen im Bereich von mehr als 10 Gew.-% benötigt werden.

In der WO 95/05164 ist die Herstellung wäßriger kolloidaler Zubereitungen von schwer wasserlöslichen Wirkstoffen wie bspw. des Ubidecarenons beschrieben, wobei die wirkstoffe in den kolloidalen Teilchen auch unterhalb ihres Schmelzpunkt im flüssigen Zustand vorliegen.

Aus der US-A 5,785,976 sind wäßrige Suspensionen kolloidaler fester Lipidpartikel, die schwerlösliche Wirkstoffe wie Ubidecarenon enthalten können, bekannt, die durch Emulgierung einer Schmelze der Wirkstoffe mit einem wäßrigen Dispersionsmedium erhalten werden.

Die WO 98/08490 offenbart feste Copräzipitate, die eine lipophite Substanz, tocopherol-polythylenglycol-succinat und eine Dispergrer lußmitell umfassen. Die lipophile substanz ist z.B. Coenzym Q10. Die Herstellung pharmazeutischer Zubereitungen nach dem Schmelzextrusionsverfahren ist an sich bekannt. So ermöglicht das beispielsweise in der EP-A 240 904 oder der EP-A 240 906 beschriebene Verfahren durch gezielte Auswahl bzw. definierte Abmischungen der eingesetzten Hilfsstoffe eine gezielte Steuerung der Eigenschaften der herzustellenden Formulierungen.

Der vorliegenden Erfindung lag die Aufgabe zugrunde, stabile, feste Zubereitungsformen für die perorale Anwendung zu finden, die insbesondere bei den schwer wasserlöslichen Ubichinonen zu einer schnellen und trotzdem langanhaltenden Solubilisierung nach deren Freisetzung (Liberation) aus der Darreichungsform dienen können.

Demgemäß wurde das Verfahren gemäß Anspruch 1 gefunden.

Als Wirkstoffe kommen wie eingangs erwähnt Ubichinone in Betracht, vor allem Ubichinon-50. Schwer löslich im erfindungsgemäßen Sinne sind Ubichinone insbesondere dann, wenn zum Lösen von 1 Teil Substanz wenigstens 100 bis 1000 Teile und vorzugsweise wenigstens 10000 Teile Wasser erforderlich sind.

Die Darreichungsform kann neben dem Ubichinon-Anteil weitere Wirkstoffe, insbesondere solche mit Ubichinon-artiger Wirkung, z.B. weitere Antioxidantien, Vitamine, wie auch Wirkstoffe anderen Typs enthalten. Eine bevorzugte Ausführungsform der vorliegenden Erfindung sind Monopräparate, die als Wirkstoffkomponente wenigstens ein Ubichinon, insbesondere Ubichinon-50 enthalten.

Die Wirkstoffkomponente macht in der Regel 1 bis 60 Gew.-%, vorzugsweise 5 bis 35 Gew.-% und insbesondere 8 bis 30 Gew.-% der Darreichungsform aus. Angaben in Gew.-% beziehen sich, sofern nicht anderes angegeben ist, auf das Gesamtgewicht der Darreichungsform.

Die erfindungsgemäßen erhältlichen Zubereitungen enthalten mindestens einen thermoplastisch verarbeitbaren Matrix-Hilfsstoff in Form wasserlöslicher pharmazeutisch akzeptabler Polymere oder Zuckeralkoholenoder Gemischen davon, sofern sie sich unzersetzt schmelzen lassen.

Matrixbildende Hilfsstoffe können insbesondere ausgewählt werden unter:

Synthetischen Polymeren, wie Polyvinyllactamen, insbesondere Polyvinylpyrrolidon (PVP); Copolymeren von Vinyllactamen, wie N-Vinylpyrrolidon, N-Vinylpiperidon und N-Vinyl-ε-caprolactam, aber insbesondere N-Vinylpyrrolidon, mit (Meth)acrylsäure und/oder (Meth)acrylsäureestern, wie langkettigen (Meth)acrylaten, z.B. Stearyl(meth)acrylat, Dialkylaminoalkyl(meth)acrylaten, die quaternisiert sein können, und Maleinsäureanhydrid, Vinylestern, insbesondere Vinylacetat, Vinylformamid, Vinylsulfonsäure oder quaternisiertem Vinylimidazol; Copolymerisaten von Vinylacetat und Crotonsäure; teilverseiftem Polyvinylacetat; Polyvinylalkohol; (Meth)acrylharzen, wie Polyhydroxyalkyl(meth)acrylaten, Poly(meth)acrylaten, Acrylatcopolymeren, z.B. aus Acrylsäurealkylestern mit (Meth)acrylsäure, und Copolymerisaten von Dimethylaminoethylacrylaten und Methacrylestern (z.B. Eudragit-Typen); Polyalkylenglykolen, wie Polypropylenglykolen und Polyethylenglykolen, vorzugsweise mit Molekulargewichten oberhalb von 1000, besonders bevorzugt oberhalb von 2000 und ganz besonders bevorzugt oberhalb von 4000 (z.B. Polyethylenglykol 6000); Polyalkylenoxiden, wie Polypropylenoxiden und vor allem Polyethylenoxiden, vorzugsweise hochmolekular, vor allem mit gewichtsmittleren Molekulargewichten von mehr als 100000; Copolymerisaten von Methylmethacrylat und Acrylsäure; Polyacrylamiden, Polyvinylformamid (gegebenenfalls partiell oder vollständig hydrolysiert);

modifizierten natürlichen Polymeren, z.B. modifizierten Stärken und modifizierten Cellulosen, wie Celluloseestern und bevorzugt Celluloseethern, z.B. Methylcellulose und Ethylcellulose, Hydroxyalkylcellulosen, insbesondere Hydroxypropylcellulose, Hydroxyalkyl-Alkylcellulosen, insbesondere Hydroxypropyl-Methylcellulose oder Hydroxypropyl-Ethylcellulose, Cellulosephthalaten, insbesondere Celluloseacetatphthalat und Hydroxypropylmethylcellulosephthalat, Stärkeabbauprodukte, insbesondere Stärkeverzuckerungsprodukte, wie Maltodextrin;

natürlichen oder überwiegend natürlichen Polymeren, wie Gelatine, Polyhydroxyalkanoaten, z.B. Polyhydroxybuttersäure und Polymilchsäure, Polyaminosäuren, z.B. Polylysin, Polyasparagin, Polydioxane und Polypeptiden, und Mannanen, insbesondere Galactomannanen; und

nichtpolymeren Bindemitteln wie Polyolen, beispielsweise den in WO 98/22094 und EP 0 435 450 beschriebenen, insbesondere Zuckeralkoholen, wie Maltit, Mannit, Sorbit, Cellobiit, Lactit, Xylit, Erythrit und Isomalt (Palatinit).

Zu den vorstehend beschriebenen Polymeren gehören beispielsweise die unter der Handelsbezeichnung Kollidon® geführten Polyvinylpyrrolidone mit gewichtsmittleren Molekulargewichten von etwa 2000 bis etwa 1,5 x 10⁶, beispielsweise das unter der Handelsbezeichnung Kollidon® 17 PF geführte Polyvinylpyrrolidon mit einem gewichtsmittleren Molekulargewicht von etwa 7000 bis etwa 11000; Vinylpyrrolidon-Vinylacetat-Copolymere insbesondere mit einem Verhältnis Vinylpyrrolidon:Vinylacetat von etwa 30 zu etwa 70 bis etwa 70 zu etwa 30, beispielsweise das unter der Handelsbezeichnung Kollidon® VA 64 geführte Produkt mit einem Verhältnis vinylpyrrolidon:Vinylacetat von etwa 60 zu etwa 40; Hydroxyalkylcellulosen mit 1 bis 3 Kohlenstoffatomen im Alkylteil, insbesondere Hydroxypropylcellulose, beispielsweise die unter der Handelsbezeichnung Klucel® geführte Hydroxypropylcellulose; Hydroxyalkyl-Alkylcellulosen mit 1 bis 3 Kohlenstoffatomen in den Alkylteilen, insbesondere Hydroxypropylmethylcellulose, beispielsweise die unter der Handelsbezeichnung Methocel® geführten Ethyl-, Hydroxyethyl-, Hydroxypropyl und Carboxymethylethergruppen enthaltenden Methylcellulose- und Methylcellulosederivatgemische, Cellulosephthalate, insbesondere Hydroxypropylmethylcellulosephthalat, Polyalkylenglycole mit 2 und/oder 3 Kohlenstoffatomen im Alkylenteil, insbesondere Polyethylenglycole, beispielsweise die unter der Handelsbezeichnung Lutrol® geführten Polyethylenglycole mit gewichtsmittleren Molekulargewichten von 2000 bis etwa 20000, und Polypropylenglycole, Copolymerisate auf Basis von Dimethylaminoethylmethacrylat und Methacrylsäureestern wie Methacrylsäuremethylester und Methacrylsäurebutylester, beispielsweise die unter der Handelsbezeichnung Eudragit® E geführten Acrylharze auf Basis von Dimethylaminoethylmethacrylat, Methyl- und Butyl(meth)acrylat mit gewichtsmittleren Molekulargewichten von etwa 150000, Copolymerisate mit anionischem Charakter auf Basis von Methacrylsäure und Methacrylsäuremethylester, beispielsweise die unter den Handelsbezeichnungen Eudragit® L bzw. S geführten Acrylharze mit gewichtsmittleren Molekulargewichten von etwa 250000 bzw. 135000.

Pharmazeutisch akzeptable Polymere sind insbesondere Homo- und Copolymere des N-Vinylpyrrolidons wie Polyvinylpyrrolidon mit K-Werten nach Fikentscher von 12 bis 100, insbesondere K 17 bis K 30, oder Copolymere mit Vinylcarbonsäureestern wie Vinylacetat oder Vinylpropionat, besonders bevorzugt Copovidone (VP/VAc-60/40).

Besonders geeigent sind auch Polyvinylalkohol oder Polyvinylacetat, welches auch verseift oder teilverseift sein kann, oder Acrylat-Polymere vom Eudragit Typ;
Cellulosederivate wie Hydroxyalkylcellulosen, beispielsweise Hydroxypropylcellulose, oder im Falle gewünschter langsamerer Freisetzung Hydroxyalkyl-alkylcellulosen, die in Wasser quellen, beispielsweise Hydroxypropyl-methylcellulose (HPMC), bevorzugt solche mit Methoxy-Substitutionsgraden im Bereich von 22 % und Hydroxypropoxy-Substitutionsgraden im Bereich von 8 %, besonders bevorzugt HPMC-Typen mit Viskositäten von 4000 mPas, 15000 mPas oder 100000 mPas, gemessen bei 20°C in 2 gew.-%iger wäßriger Lösung. Geeignet sind auch HPMC-typen mit Methoxy-Substitutionsgraden im Bereich von 28 bis 29 % und Hydroxypropoxy-Substitutionsgraden im Bereich von 5 bis 8,5 %;
schmelzbare Zuckeralkohole wie beispielsweise Sorbit, Maltit, Isomalt, Mannit, Xylit, Erythrit oder Mischungen daraus, wobei Maltit, Mannit, Xylit und/oder Isomalt bevorzugt sind;
Polyethylenglykole mit Molekulargewichten im Bereich von 1000 bis 20000000 Dalton, bevorzugt 4000 bis 10000 Dalton.

Der Anteil matrixbildender Hilfsstoffe an der Dareichungsform beträgt in der Regel 20 bis 95 Gew.-%, vorzugsweise 30 bis 90 Gew.-% und insbesondere 40 bis 80 Gew.-%.

Als oberflächenaktive Substanzen kommen niedermolekulare Substanzen, also nicht-polymere Verbindungen, in Betracht, die einen HLB-Wert (HLB - Hydrophilic Lipophilic Balance von 2 bis 18 aufweisen, und bei 20°C flüssig sind oder einen Tropfpunkt im Bereich von 20°C bis 50°C aufweisen, bevorzugt von bis zu 40°C. Bevorzugt werden Substanzen mit einem HLB-Wert von 7 bis 18, besonders bevorzugt 10 bis 15. Das HLB-System (Hydrophil-Lipophil-Balance system) ordnet grenzflächenaktiven Stoffen numerische Zahlenwerte zu, lipophile Substanzen erhalten niedrige, hydrophile höhere HLB-Werte (Fiedler, H.B., Lexikon der Hilfsstoffe für Pharmazie, Kosmetik, und angrenzende Gebiete, 4.Aufl., Aulendorf: ECV-Editio-Cantor-Verlag (1996)).

Geeignete oberflächenaktive Substanzen sind beispielsweise gesättigte und ungesättigte polyglykolisierte Glyceride, halbsynthetische Glyceride, Fettsäurester oder Ether von Fettsäurealkoholen, sofern sie die oben angegebenen Eigenschaften aufweisen. Besonders geeignet ist auch die Ölsäure.

Der Begriff Fettsäure bezeichnet eine Gruppe aliphatischer gesättigter oder ungesättigter Carbonsäuren. In der Regel sind es unverzweigte Ketten mit 6 bis 30, vorzugsweise 8 bis 22 und insbesondere 8 bis 18 Kohlenstoffatomen. Zu den gesättigten Fettsäuren gehören beispielsweise Capronsäure, Önanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Behensäure, Lignocerinsäure, Cerotinsäure und Melissinsäure. Die ungesättigten Fettsäuren können einfach oder mehrfach ungesättigt, insbesondere einfach, zweifach, dreifach, vierfach, fünffach oder sechsfach ungesättigt sein. Beispielsweise gehören zu den einfach ungesättigten Fettsäuren Palmitoleinsäure, Ölsäure und Erucasäure, zu den zweifach ungesättigten Fettsäuren Sorbinsäure und Linolsäure, zu den dreifach ungesättigten Fettsäuren Linolensäure und Elaeostearinsäure, zu den vierfach ungesättigten Fettsäuren Arachidonsäure, zu den fünffach ungesättigten Fettsäuren Clupanodonsäure und zu den sechsfach ungesättigten Fettsäuren Docosahexaensäure. Ebenfalls zu den Fettsäuren im erfindungsgemäßen Sinn gehören Fettsäurederivate, wie hydroxylierte Fettsäuren, z.B. Hydroxystearinsäure, Dihydroxystearinsäure und Trihydroxystearinsäure.

Mit dem Begriff Glyceride bezeichnet man Ester des Glycerins. Je nach Anzahl der Estergruppen spricht man von Mono-, Di- und Triglyceriden. Der Säurerest eines Monoglycerids kann an 1- oder 2-Position sitzen und die Säurereste von Di- und Triglyceriden können gleich oder verschieden sein und in jeder erdenklichen Art auf die drei möglichen Positionen des Glycerins verteilt sein. Bei den Säureresten handelt es sich bevorzugt um die zuvor beschriebenen Fettsäuren. Beispielsweise gehören zu den Monoglyceriden Glycerinmonobehenat, Glycerinmonocaprat, Glycerinmonococoat, Glycerinmonoerucat, Glycerinmonoisostearat, Glycerinmonolanolat, Glycerinmonolaurat, Glycerinmonolinoleat, Glycerinmonomyristat, Glycerinmonooleat, Glycerinmonopalmitat, Glycerinmonoricinoleat, Glycerinmonostearat, zu den Diglyceriden Glycerindicaprylat, Glycerindilaurat, Glycerindimyristat, Glycerindioleat, Glycerindipalmitat und Glycerindistearat; zu den Triglyceriden Glycerintricaprylat, Glycerintrilaurat, Glycerintrimyristat, Glycerintrioctanoat, Glycerintrioleat, Glycerintriricinoleat und Glycerintristearat.

Zu den oben genannten Fettsäureestern gehören vor allem solche der Formel II,

[R-CO-(EO)ₓ-]_{y}A (II)

wobei R für einen geradkettigen oder verzweigten, gesättigten oder ungesättigten, gegebenenfalls mono-, di- oder trihydroxylierten aliphatischen Rest mit 8 bis 30 Kohlenstoffatomen steht, die Summe aller x Null bis 100 beträgt, y für 1 bis 7 steht, A für Hydroxy oder C₁-C₈-Alkyloxy steht, wenn y für 1 steht, oder A von einem Polyol abgeleitetet ist, wenn y für 2 bis 7 steht.

Der Rest R leitet sich von Fettsäureresten ab, beispielsweise den oben genannten, so daß R zweckmäßigerweise für einen geradkettigen oder - insbesondere 1- bis 2-fach - verzweigten, gesättigten oder - insbesondere einfach, zweifach oder dreifach - ungesättigten, gegebenenfalls mono-, di- oder trihydroxylierten aliphatischen Rest mit 8 bis 30, vorzugsweise 12 bis 24 und insbesondere 10 bis 24 Kohlenstoffatomen steht. Insbesondere gehören hierzu Palmityl, Stearyl, Arachidyl, Hexadecenyl, Oleyl, Linolyl, Linolenyl, Rizinoleyl, Eiocosanyl sowie Mono- und Dihydroxystearyl, von denen Rizinoleyl und Oleyl besondere Bedeutung besitzen.

Sind die nichtionischen Tenside der Formel II ethoxyliert, so gibt die Summe aller x die mittlere Ethoxylierungszahl mit in der Regel 3 bis 100 und insbesondere 4 bis 50 an.

Steht A für Hydroxy, so ist y = 1. Insbesondere sind in diesem Zusammenhang entsprechende Rhizinusöl- und Ölsäurepolyethoxylate zu nennen.

Steht A für gegebenenfalls verzweigtes Alkyloxy, vorzugsweise mit 1 bis 4 und vorzugsweise 1 oder 2 Kohlenstoffatomen, so ist y = 1.

Ist A von einem Polyol mit 3 bis 7 und insbesondere 6 Kohlenstoffatomen abgeleitet, so beträgt der Wert von y = 2 bis 7 und vorzugsweise 3 bis 6. Dabei sind y Hydroxywasserstoffatome des Rests A durch jeweils einen Rest R-CO-(EO)ₓ- ersetzt, wobei mehrere Reste R und mehrere Indizes x gleich oder verschieden sein können. Vorzugsweise sind mehrere Reste R gleich, während die Indizes x verschieden sein können und in der Regel einer Gauß-Verteilung folgen. Insbesondere leitet sich A von einem Zuckeralkohol, wie Sorbitol oder Glycerin ab.

Insbesondere eignen sich die entsprechenden Sorbitanfettsäureester oder ethoxylierte Sorbitanfettsäureester
wie beispielsweise Polyoxyethylen(20)sorbitanmonolaurat,
Polyoxyethylen(20)sorbitanmonopalmitat,
Polyoxyethylen(20)sorbitanmonostearat,
Polyoxyethylen(20)sorbitanmonooleat,
Polyoxyethylen(20)sorbitantristearat,
Polyoxyethylen(20)sorbitantrioleat,
Polyoxyethylen(4)sorbitanmonostearat,
Polyoxyethylen(4)sorbitanmonolaurat oder
Polyoxyethylen(4)sorbitanmonooleat.
Weiterhin eignen sich Macrogol-6-Cetylstearylether oder Macrogol-25-Cetylstearylether.

Besonders bevorzugt sind Polyoxyethylenglycerolricinolat-35, Polyoxyethylenglyceroltrihydroxystearat-40, PEG-660-12-Hydroxystearinsäure (Polyglykolester der 12-Hydroxystearin-säure (70 mol-%) mit 30 mol-% Ethylenglykol).

Besonders bevorzugt ist weiterhin Vitamin E-polyethylenglycol-1000-succinat (auch als Vitamin-E-TPGS bekannt). Da dieses Vitamin-E-Derivat auch gleichzeitig zur Nahrungsergänzung eingesetzt wird, bieten Darreichungsformen, die neben dem Ubichinon-Wirkstoff auch Vitamin-E-TPGS enthalten den Vorteil, daß sie eine Kombinationsbehandlung ermöglichen.

Die oberflächenaktiven Substanzen sind in den Zubereitungen in Mengen von mehr als 1 Gew.-%, bezogen auf das Gesamtgewicht der Darreichungsform und insbesondere der festen Dispersion, also der Gesamtmenge aus Wirkstoff, matrixbildendem Hilfsstoff und weiteren Hilfsstoffen, und bis zu 40 Gew.-% enthalten, bevorzugt 2 bis 25 Gew.-% und besonders bevorzugt 20 bis 25 Gew.-%.

Weiterhin können die Zubereitungen noch übliche pharmazeutische Hilfsstoffe wie Aromen, Antioxidantien, Kieselsäuren, Trennmittel oder Farbstoffe in den hierfür üblichen Mengen enthalten. Bevorzugt enthalten die Zubereitungen hochdisperse Kieselsäuren. Es kann sich auch empfehlen, Weichmacher, bevorzugt Triethylcitrat, zuzusetzen.

Die Herstellung der erfindungsgemäß erhältlichen Zubereitungen erfolgt über ein Schmelzeverfahren. Das Verfahren wird ohne Zugabe von Lösungsmitteln durchgeführt.

Das Schmelzeverfahren wird in einem Kneter oder einem Schneckenextruder durchgeführt. Geeignete Kneter sind beispielsweise Kneter der Firmen Haake, Buss, List oder Farrell.

Bevorzugt erfolgt die Herstellung der Schmelze in einem Schneckenextruder, besonders bevorzugt einem Doppelschneckenextruder mit und ohne Knetscheiben oder ähnlichen Mischelementen. Besonders bevorzugt sind gleichsinnig drehende Doppelschneckenextruder.

In Abhängigkeit von der Zusammensetzung erfolgt die Verarbeitung im allgemeinen bei Temperaturen von 40°C bis 150°C, bevorzugt 50 bis 120°C. Die Verweilzeiten liegen in der Regel im Minutenbereich.

Die Einsatzstoffe können dem Extruder oder Kneter einzeln oder als Vormischung zugeführt werden. Die Zugabe erfolgt bevorzugt in Form von pulverförmigen oder granulierten Vormischungen. So kann die flüssige oder ölige oberflächenaktive Substanz zuvor mit einem anderen Einsatzstoff zu einem rieselfähigen Granulat vermengt werden. Eine Zugabe der oberflächenaktiven Substanz in flüssiger Form, beispielsweise über Flüssigkeitspumpen, die bei halbfesten Substanzen vorzugsweise beheizt werden, ist ebenfalls möglich.

Man kann auch zunächst den Wirkstoff in der oberflächenaktiven Substanz lösen und diese Mischung dann mit dem Polymer granulieren. Dabei muß der Wirkstoff selbst nicht schmelzen.

Es kann sich auch empfehlen, zunächst die anderen Einsatzstoffe aufzuschmelzen und dann erst den Wirkstoff zuzugeben.

Die Einsatzstoffe werden demgemäß gemeinsam zu einer Schmelze verarbeitet, welche durch Eintragen mechanischer Energie, insbesondere in Form von Scherkräften, zu einer homogenen Masse verarbeitet wird.

Die homogene Schmelze wird anschließend durch eine Düse oder eine Lochplatte extrudiert und der Formgebung unterworfen. Dies kann durch Abschlag des strangförmig austretenden Extrudats mit den üblichen Abschlagtechniken erfolgen, beispielsweise mit Hilfe rotierender Messer oder durch Druckluftabschlag, wobei Pellets oder Granulate entstehen. Weiterhin kann die Formgebung wie in der EP-A 240 906 beschrieben erfolgen, indem das strangförmig austretende Extrudat zwischen zwei gegenläufig rotierende Kalanderwalzen geführt und direkt zu Tabletten ausgeformt wird. Ebenso kann die Schmelze über den offenen Extruderkopf ausgefahren werden und nach Erstarren gegebenenfalls noch gemahlen werden oder durch geeignete Granuliergeräte wie Walzenstühle oder Kompaktiereinheiten weiterverarbeitet werden.

Die erstarrten Schmelzen sind Festkörper, in denen der Wirkstoff bevorzugt molekulardispers in der Hilfsstoffmatrix verteilt vorliegt.

Auch die erfindungsgemäß erhältlichen Darreichungsformen sind bevorzugt fest. Somit kann es sich insbesondere µm Pulver, Granulate, Pellets oder Tabletten handeln.

Granulate oder Pellets können in konventionellen Tablettenpressen zu Tabletten verarbeitet werden. Es ist auch möglich, die durch Kalandrierung zunächst bereits in Form von mechanisch stabile Tabletten erhaltenen Zubereitungen einem Mahlvorgang zu unterwerfen und dann auf konventionelle Weise zu Tabletten zu verpressen. Gewünschtenfalls können die Tabletten dann mit einem üblichen Überzug versehen werden.

Überraschenderweise lassen sich erfindungsgemäß Darreichungsformen und insbesondere Tabletten erhalten, die trotz eines hohen Anteils an flüssigen oder halbfesten oberflächenaktiven Substanzen stabil sind. Insbesondere weisen sie eine gute mechanische Stabilität, d.h. vor allem Formstabilität, auf, so daß sie vor allem nicht zur Klebrigkeit und/oder zum Erweichen neigen. Die Formen sind bei Raumtemperatur lagerstabil. Dies war im Hinblick darauf, daß nicht nur die oberflächenaktiven Substanzen, sondern auch die Ubichinone niedrigschmelzend sind, nicht zu erwarten. Auf eine Abfüllung in Kapseln kann somit verzichtet werden.

Es war auch nicht zu erwarten, daß sich terpenoide Systeme, die zusätzlich eine so empfindliche Gruppierung wie die Chinon-Funktion enthalten, nahezu unzersetzt auch bei Temperaturen über der Schmelztemperatur verarbeiten ließen.

Die resultierenden Darreichungsformen enthalten den Wirkstoff insbesondere als amorphe Einbettung. Bevorzugt entstehen feste Dispersionen, in denen der Wirkstoffe molekulardispers verteilt vorliegt. Die erfindungsgemäß erhältlichen Darreichungsformen ermöglichen es auch, die schwerlöslichen Ubichinone ausreichend zu solubilisieren bzw. in wäßrigem Medium stabil zu dispergieren. Es war nicht zu erwarten, daß die erfindungsgemäß erhältlichen Darreichungsformen trotz des stark lipidischen Charakters der Ubichinone selbstdispergierend wären.

Der Begriff "molekulardispers" ist dem Fachmann bekannt und beschreibt im Wesentlichen Systeme, in denen eine Substanz, im vorliegenden Fall wenigstens ein Teil und vorzugsweise der überwiegende Teil des Ubichinon-Anteils, in einem Bindemittel in homogener Verteilung dispergiert ist. In der molekulardispersen Verteilung ist das System grenzflächenfrei. Das Bindemittel bildet dabei in der Regel eine Matrix, die erfindungsgemäß von der Bindemittelkomponente oder zumindest von einem überwiegenden Teil der Bindemittelkomponente gebildet wird.

Der Anteil an Wirkstoffkristallen in einer erfindungsgemäß erhältlichen Formulierung liegt in der Regel unter 12% und insbesondere unter 5%. Angaben zu Kristallanteilen beziehen sich auf die Gesamtmenge des oder der Wirkstoffe, insbesondere des Ubichinon-Anteils.

Eine erfindungsgemäß erhältliche Formulierung, die im Wesentlichen frei von Wirkstoffkristallen ist, stellt eine besondere Ausführungsform der vorliegenden Erfindung dar. Mit der Verminderung des Kistallanteils erhöht sich die Homogenisierung des Wirkstoffs in der Matrix.

Erfindungsgemäß erhältliche Formulierungen, in denen im Wesentlichen kein Bestandteil kristalline Anteile aufweist (im wesentlichen amorphe bzw. kristallfreie Formulierungen), stellen eine weitere besondere Ausführungsform der vorliegenden Erfindung dar.

Der Zustand solcher molekulardispersen Verteilungen läßt sich mit bekannten analytischen Verfahren untersuchen, z.B. mit der Differential Scanning Calorimetry (DSC) oder mit Weitwinkelröntgen-streuung-Messungen (WAXS-Messungen). Liegt eine molekulardisperse Verteilung vor, so fehlt der bei der DSC-analytischen Messung der kristallinen Reinsubstanz auftretende, in der Regel endotherme Schmelzpeak. Eine weitere Möglichkeit zur Kennzeichnung einer molekulardispersen Verteilung ist die Intensitätsverminderung und/oder Abwesenheit typischer Röntgenbeugungs-Signale bei WAXS-Analytik.

Die erfindungsgemäß erhältlichen Zubereitungen bilden nach Auflösen in wäßrigem Medium, insbesondere bei pH-Werten von 1, für mindestens eine Stunde ein stabiles Solubilisat oder eine stabile Dispersion, in der Wirkstoff bevorzugt nicht-kristallin vorliegt.

Die erfindungsgemäß erhältlichen Formen eignen sich zur Verwendung als Nahrungergänzungsmittel, diätetische Mittel sowie als Arzneimittel.

### Allgemeine Vorschrift:

Die oberflächenaktive Substanz und das Matrixpolymer wurden zunächst vorgranuliert, indem man das pulverförmige Polymer mit der flüssigen bzw. halbfesten oberflächenaktiven Substanz verknetete, so daß ein homogenes, rieselfähiges Granulat entstand. Danach wurden Ubichinon und hochdisperse Kieselsäure zugemischt. Diese Mischung wurde dann über eine Dosierwaage in einen Schmelze-Extruder dosiert. Die Verfahrens-Parameter der Extrusion wurden so eingestellt, daß die Materialtemperatur der Schmelzen zwischen 80 und 100°C betrugen. Die austretenden Produkte stellten orangefarbene, je nach verwendetem Hilfsstoff, mehr oder weniger klare Schmelzen dar, die auch nach dem Abkühlen ihr Aussehen nicht veränderten. Erkaltete Schmelzestücke lösten sich bei 37°C in Wasser unter Ausbildung opaker kolloidaler Lösungen.

| Beispiel Nr. | 1 | 2 | 3 |
|---|---|---|---|
| Ubichinon-50 | 20 | 20 | 20 |
| VA 64¹⁾ | 67 | 67 | 67 |
| Cremophor RH40²⁾ | 12 | | |
| Vitamin-E-TPGS³⁾ | | 12 | |
| Ölsäure | | | 12 |
| Aerosil 200⁴⁾ | 1 | 1 | 1 |

| | | | |
|---|---|---|---|
| ¹⁾Copolymer aus 60 Gew.-% N-Vinylpyrrolidon/40 Gew.-% Vinylacetat ²⁾Polyoxyethylentrihydroxystearat 40 ³⁾Vitamin-E-polyethylenglykol-1000-succinat ⁴⁾ Hochdisperse Kieselsäure, BET-Oberfläche 200 + 25 | | | |

## Patentansprüche

1. Verfahren zur Herstellung stabiler Darreichungsformen zur peroralen Verabreichung, enthaltend
a) ein Ubichinon als Wirkstoff,
b) mindestens einen thermoplastisch verarbeitbaren matrixbildenden Hilfsstoff in Form wasserlöslicher Polymere oder Zuckeralkoholen oder Gemischen daraus, und
c) 1 bis 40 Gew.-%, bezogen auf das Gesamtgewicht der Darreichungsform, einer oberflächenaktiven Substanz mit einem HLB-Wert von 2 bis 18, welche bei 20 °C flüssig ist oder einen Tropfpunkt im Bereich von 20 bis 50 °C aufweist, oder Ölsäure,
nach einem Schmelzverfahren, wobei man die Einsatzstoffe in der Schmelze ohne Zugabe von Lösungsmitteln zu einer homogenen Mischung verarbeitet und diese zu Darreichungsformen ausformt.

2. Verfahren nach Anspruch 1, wobei die Darreichungsformen als matrixbildenden Hilfsstoff ein Copolymer des N-Vinylpyrrolidons enthalten.

3. Verfahren nach Anspruch 1 oder 2, wobei die Darreichungsformen 2 bis 25 Gew.-% an oberflächenaktiver Substanz enthalten.

4. Verfahren nach einem der Ansprüche 1 bis 3, wobei die Darreichungsformen oberflächenaktive Substanzen mit einem HLB-Wert von 10 bis 15 enthalten.

5. Verfahren nach einem der Ansprüche 1 bis 4, wobei die Darreichungsformen oberflächenaktive Substanzen mit einem Tropfpunkt im Bereich von 20 bis 40 °C enthalten.

6. Verfahren nach einem der Ansprüche 1 bis 5, wobei die Darreichungsformen als oberflächenaktive Substanzen Macrogol-glycerol-hydroxy-stearat, Polyoxyethylenricinoleat-35 oder PEG-660-12-Hydroxystearinsäure enthalten.

7. Verfahren nach einem der Ansprüche 1 bis 6, wobei die Darreichungsformen als oberflächenaktive Substanz ein Vitamin-E-polyoxyethylen-glykol-1000-succinat enthalten.

8. Verfahren nach einem der Ansprüche 1 bis 7, wobei die Darreichungsformen als Wirkstoff Ubichinon-50 enthalten.

## Claims

1. A process for producing stable dosage forms for oral administration, comprising
a) a ubiquinone as active ingredient,
b) at least one melt-processable matrix-forming excipient in the form of water-soluble polymers or sugar alcohols or mixtures thereof, and
c) 1 to 40% by weight, based on the total weight of the administration form, of a surface-active substance with an HLB of from 2 to 18, which is liquid at 20°C or has a drop point in the range from 20 to 50°C, or oleic acid,
by a melt process, the starting materials being processed in the melt without the addition of solvents to give a homogeneous mixture, and the latter being shaped to give dosage forms.

2. The process according to claim 1, the dosage forms comprising as matrix-forming excipient a copolymer of N-vinylpyrrolidone.

3. The process according to claim 1 or 2, the dosage forms comprising 2 to 25% by weight of surface-active substance.

4. The process according to any of claims 1 to 3, the dosage forms comprising surface-active substances with an HLB of from 10 to 15.

5. The process according to any of claims 1 to 4, the dosage forms comprising surface-active substances with a drop point in the range from 20 to 40°C.

6. The process according to any of claims 1 to 5, the dosage forms comprising as surface-active substances macrogol glycerol hydroxystearate, polyoxyethylene ricinoleate 35 or PEG 660 12-hydroxystearate.

7. The process according to any of claims 1 to 6, the dosage forms comprising as surface-active substance a vitamin E polyoxyethylene glycol 1000 succinate.

8. The process according to any of claims 1 to 7, the dosage forms comprising as active ingredient ubiquinone 50.

## Revendications

1. Procédé de préparation de formes galéniques stables pour administration orale, contenant
a) une ubiquinone comme agent actif,
b) au moins un auxiliaire thermoplastique, formant matrice, sous forme de polymères solubles dans l'eau ou d'alcools sacchariques ou de leurs mélanges, et
c) 1 à 40% en poids, sur base du poids total de la forme galénique, d'une substance tensioactive avec une valeur EHL allant de 2 à 18, qui est liquide à 20°C ou qui présente un point de suintement situé dans l'intervalle allant de 20 à 50°C, ou l'acide oléique,
selon un procédé à l'état fondu, où l'on traite les substances mises en oeuvre dans la masse fondue sans addition de solvant, en un mélange homogène, et l'on forme celui-ci en les formes galéniques.

2. Procédé selon la revendication 1, où les formes galéniques contiennent comme auxiliaire formant matrice un copolymère de N-vinylpyrrolidone.

3. Procédé selon la revendication 1 ou 2, où les formes galéniques contiennent 2 à 25% en poids de substances tensioactives.

4. Procédé selon l'une des revendications 1 à 3, où les formes galéniques contiennent des substances tensioactives ayant une valeur EHL allant de 10 à 15.

5. Procédé selon l'une des revendications 1 à 4, où les formes galéniques contiennent des substances tensioactives ayant un point de suintement situé dans l'intervalle allant de 20 à 40°C.

6. Procédé selon l'une des revendications 1 à 5, où les formes galéniques contiennent comme substances tensioactives le Macrogol-hydroxystéarate de glycérol, le ricinoléate de polyéthylène-35 ou le PEG-660-acide 12-hydroxystéarique.

7. Procédé selon l'une des revendications 1 à 6, où les formes galéniques contiennent comme substances tensioactives un succinate de vitamine E-polyoxyéthylène-glycol 1000.

8. Procédé selon l'une des revendications 1 à 7, où les formes galéniques contiennent comme agent actif l'ubiquinone-50.
